# EUROPEAN PATENT SPECIFICATION

(11) **EP 2 868 324 B1**
(45) Date of publication and mention of the grant of the patent: **08.04.2020**
(21) Application number: 12832703.8
(22) Date of filing: 28.06.2012
(51) Int. Cl.: A61K 36/53, A61K 8/67, A61K 8/97, A61K 36/00, A61P 37/08, A61Q 19/00, A61Q 19/08

(54) **HYALURONIC ACID DECOMPOSITION INHIBITOR COMPRISING ROSEMARY EXTRACT AND RETINOL ACETATE**
HYALURONSÄURE-ZERSETZUNGSHEMMER MIT EINEM ROSMARINEXTRAKT UND RETINOLACETAT
INHIBITEUR DE DÉCOMPOSITION D'ACIDE HYALURONIQUE COMPRENANT DE L'EXTRAIT DE ROMARIN ET DE L'ACÉTATE DE RÉTINOL

(43) Date of publication of application: 06.05.2015
(73) Proprietor: Shiseido Company, Ltd., Tokyo 104-0061 (JP)
(72) Inventor: IINO, Masato, Yokohama-shi Kanagawa 224-8558 (JP); OCHIAI, Nobuhiko, Yokohama-shi Kanagawa 224-8558 (JP); TSUNENAGA, Makoto, Yokohama-shi Kanagawa 224-8558 (JP)
(74) Representative: Santarelli
(86) International application number: PCT/JP2012/066593
(87) International publication number: WO 2014/002232

(56) References cited:
- WO-A1-01/30314
- WO-A1-2009/119099
- WO-A2-02/47641
- WO-A2-2006/029818
- FR-A1- 2 260 326
- JP-A- H1 095 735
- JP-A- H08 333 267
- JP-A- 2002 080 338
- JP-A- 2006 241 044
- JP-A- 2007 508 320
- JP-A- 2010 270 062
- RO-B1- 120 383
- US-A1- 2007 003 536
- DATABASE GNPD [Online] MINTEL; 30 September 2010 (2010-09-30), "Face Cream", XP002746155, Database accession no. 1412041
- IPPOUSHI, K. ET AL.: 'Evaluation of inhibitory effects of vegetables and herbs on hyaluronidase and identification of rosmarinic acid as a hyaluronidase inhibitor in lemon balm (Melissa officinalis L.)' FOOD SCIENCE AND TECHNOLOGY RESEARCH vol. 6, no. 1, 2000, pages 74 - 77, XP055136215
- YOSHIHITO FUJIWARA ET AL.: 'Chemical constituents and physiological activity of Rosmarinus officinalis L.' CSJ: THE CHEMICAL SOCIETY OF JAPAN KOEN YOKOSHU vol. 72, no. 2, 1997, page 1419, XP008171607

## Description

### [Technical Field]

The present invention relates to an inhibitor for hyaluronidase which has a good hyaluronidase inhibitory activity.

### [Background Art]

Recently, research studies relating to aging have been conducted. In a macroscopic aspect, increased age is a major cause for skin aging. In addition to increased age, the influences resulted from drying, oxidizing, and sunlight (ultraviolet rays) were also known as a direct cause for skin aging. A reduction of mucopolysaccharides, such as hyaluronic acid, etc., cross-linking reaction of collagen, and cellular damage due to ultraviolet rays were known as a concrete phenomenon of skin aging.

Inter alia, hyaluronic acid, one of glycosaminoglycans, which is widely present in the connective tissue of mammals along with chondroitin sulfate, etc., and is a linear high molecular polysaccharide comprising β-D-N-acetylglucosamine and β-D-glucuronic acid alternately bound to each other, has various functions, such as retaining water in the intercellular space, holding cells by forming a gelatinous matrix in the tissue, maintaining lubricity and flexibility of the tissue, resisting against external force applied, such as mechanical stress, and inhibiting cellular infection (See, NPL 1). In addition, the amount of hyaluronic acid is decreased with increasing age, thereby skin aging, such as a finely wrinkled skin or dry and rough skin is thought to appear. Therefore, many cosmetics comprising hyaluronic acid are provided as an aged skin improving agent. However, such previous cosmetics cannot improve the aged skin fundamentally, but merely exert a moisture retention effect on the skin surface. Although several cosmetics comprising an agent that increases the amount of hyaluronic acid in the skin have been recently provided (see, PLT1), such cosmetics are not a product which can clearly improve and treat the aged skin. It was believed that the aged skin cannot necessarily be improved by externally applying hyaluronic acid or an agent which accelerates production of hyaluronic acid to the skin, since hyaluronic acid degrades promptly in the skin. Once dermal hyaluronic acid is synthesized in vivo, it is immediately subjected to the enzymatic degradation from hyaluronidase, a degradative enzyme. Therefore, the degradation rate is high, and the half-life of hyaluronic acid is reported to be about one day (see, NRP2). Hyaluronidase is an enzyme which can degrade hyaluronic acid as speculated from its name, and is distributed widely in the living body and also present in the skin. Therefore, it was believed that the inhibition of the activity of hyaluronidase, which promotes the degradation of hyaluronic acid, can contribute to the stability of hyaluronic acid used in a formulation, and also to the stability of hyaluronic acid originally present in the skin or derived from the formulation after applying it to the skin. In addition, since hyaluronidase was also known as an inflammatory enzyme, inhibiting the activity thereof can inhibit inflammation, and can also repress allergy. Due to the above reasons, in order to maintain skin elasticity and moisture retaining property, thereby preventing wrinkled, finely wrinkled and dry-and-rough skin and maintaining a moist and fresh skin condition, the development of an effective inhibitor for hyaluronidase, a hyaluronic acid degrading enzyme, has been required.

Extract of a plant of the Labiatae family, such as rosemary, thyme, and melissa, etc., has been shown to inhibit hyaluronidase, suppress platelet aggregation, and suppress exosaminidase liberation, and therefore the extract are shown to have an anti-inflammatory effect (PLT2). A hyaluronidase inhibitory substance extracted from any one of rosemary, thyme, or melissa, a mixture thereof, and a mixture further comprising an anti-allergic agent were described in the document. However, there was no description relating to a mixture which can exert a synergic effect over an additive effect.

### [Citation List]

### [Patent Literature]

PLT1: Japanese Unexamined Patent Publication (Kokai) No. 11-209261
PLT2: Japanese Unexamined Patent Publication (Kokai) No. H08-333267

### [Non patent literature]

NPL1: "BioIndustry", vol.8, p.346 (1991)
NPL2: Tammi R. et al., J. Invest. Dermatol., 97, 126-130 (1991)

Record ID 1412041 of the GNPD Mintel database cites a face cream comprising retinyl palmitate in combination with (inter alia) Rosmarinus Officinalis Extract.

### [Summary of Invention]

### [Technical Problem]

The present inventions are premised on the above circumstance, and are intended to provide a hyaluronidase inhibitor having a hyaluronidase inhibitory activity in the human body which is safe and can be easily used.

### [Solution to Problem]

In order to solve the above problem, the inventors have carried out extensive researches for screening widely various materials on a hyaluronidase inhibitory activity, and surprisingly found that a hyaluronidase activity, and surprisingly found that a hyaluronidase inhibitory activity is drastically enhanced when rosemary extract, which is known to exert a hyaluronidase inhibitory activity, is used in combination with Vitamin A, which is not known to exert a hyaluronidase inhibitory activity. Based on this discovery, the inventors have completed the present invention.

Accordingly, the present invention is as laid out in the claims.

### [Advantageous Effects of Invention]

When vitamin A, which is included in a hyaluronidase inhibitor of the present invention, is used alone, a hyaluronidase inhibitory activity is not exerted, or is hardly exerted. However, when vitamin A is used in combination with rosemary extract, vitamin A significantly enhances the hyaluronidase inhibitory activity of rosemary extract. Therefore, the hyaluronidase inhibitor of the present invention exerts either effects of improving or preventing skin problems, such as human skin aging, wrinkled skin, rough skin, dry skin, dry-and-rough skin, acne, and atopic dermatitis, or improving or preventing disease related to a reduction in hyaluronic acid, such as arthritis, gingivitis, rheumatism, osteoarthritis, or burn injury, etc.

### [Brief Description of Drawings]

[Fig. 1]

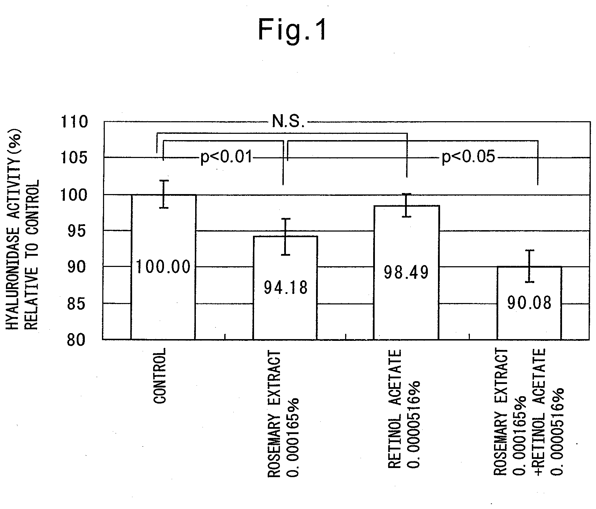

Fig. 1 shows that while rosemary extract has a hyaluronidase activity, but retinol acetate does not have a hyaluronidase activity, the hyaluronidase inhibitory activity of rosemary extract can be significantly enhanced by combined use of rosemary extract and retinol acetate.

### [Description of Embodiments]

In the present invention, hyaluronidase is a collective term of an enzyme which degrades hyaluronic acid by cleaving a glucoside linkage. Since hyaluronidase is widely distributed in the mammal tissue, the amount of hyaluronic acid inside the body, in particular inside the skin or joint fluid, is expected to be increased by inhibiting the activity of hyaluronidase. Glycoside linkage to be cleaved can be glucronide linkage or N-acetylglucosaminide linkage.

In the present invention, a hyaluronidase inhibitor is an agent inhibiting a hyaluronic acid degradation activity of hyaluronidase, and therefore it can increase the amount of hyaluronic acid inside the body, in particular joint fluid or skin. In a preferable aspect of the present invention, a hyaluronidase inhibitor includes rosemary extract and vitamin A.

Hyaluronic acid relates to elasticity, moisture retaining property, and lubricity inside the body. It is also known that the amount of hyaluronic acid diminishes with aging, and that symptoms, such as wrinkled skin, rough skin, dry skin or dry-and-rough skin appear due to a reduction in hyaluronic acid in the skin. In addition, it is also known that hyaluronidase relates to inflammatory, and that the activity of hyaluronidase relates to atopic dermatitis or acne. Therefore, the skin problems, such as aging, wrinkled skin, rough skin, dry skin, dry-and-rough skin, acne, or atopic dermatitis, and diseases related to a reduction in hyaluronic acid, such as dry skin, arthritis, gingivitis, rheumatism, osteoarthritis, and burn injury can be treated, improved or prevented by the hyaluronidase inhibitor of the present invention.

In the present invention, rosemary extract means an extract derived from Lamiaceae (family), Rosmarinus (genus) rosemary (Rosmarinus officinalis), which is indigenous to the Mediterranean region. All parts of rosemary, in particular the whole plant, leaf, and flower can be used as a source material. Rosemary extract is known to have an antibiotic property, antioxidant property, and anti-inflammatory property, and therefore it was reported that rosemary extract can be used as a pharmaceutical product, such as an anti-aging agent, such as a skin improvement agent; an immunomodulatory agent; an anti-diabetic agent; an anti-osteoporotic agent; an anti-adiposity agent; a sleep enhancing agent; anti-central nervous agent; etc.

The rosemary extract of the present invention can be prepared in accordance with a known method, for example by immersing the plant in extracting solvent, or heating to reflux the solvent, and then carrying out a filtration and condensation. Any extracting solvents usually used in an extraction can be used. For example, water, alcohols, such as methanol, ethanol, propylene glycol, 1,3-buthyleneglycol, glycerine, hydroalcoholic solutions, chloroform, dichlorethane, carbon tetrachloride, acetone, ethyl acetate, hexane, etc., can be used independently or in combination as a extracting solvent. An extract obtained by extracting with the above solvent can be used without purification. Alternatively, an extract which is subjected to an adsorption method using, for example, an ionic exchange resin, so as to remove impurities, or which is subjected to a porous polymer column (for example Amberlite XAD-2) to adsorb thereon, eluted with methanol or ethanol, and is then concentrated, can be used. A dried extract can also be used. In addition, an extract which is extracted according to a distribution method, for example by using water/ethyl acetate, can also be used.

The extract of the plant obtained thereby has a good hyaluronidase inhibitory activity. Since the extract is used as a cosmetic or pharmaceutical product, which is directly applied to the skin, such as a skin external agent, etc., the extract extracted with water, 1,3-butylene glycol, glycerine, etc., is preferable. For example, the extract extracted prepared by means of 50 w/w% 1,3-butyleneglycol aqueous solution is more preferable.

When the above plant or its extract is contained in a cosmetic or pharmaceutical product, it is contained in the cosmetic or pharmaceutical product at 0.00001 to 0.1 w/w%, preferably 0.00005 to 0.01 w/w%, more preferably 0.0001 to 0.001 w/w%, at dry weight.

A deficiency of Vitamin A is known to induce night blindness or rachitis, and relating to the skin, also known to result in keratinization of skin or mucosa, abnormal dryness of skin, and pigment deposition. Therefore, vitamin A has been used for skin in order to treat acne or promote skin turn over. However, it is not described nor suggested that vitamin A enhances a hyaluronidase inhibitory activity. Vitamin A includes one or more ingredients selected from vitamin A group, such as retinol, retinal, retinoic acid, and a precursor or derivative thereof, such as retinol acetate, retinol palmitate, tretionic acid, methyl retinate, ethyl retinate, retinoic acid retinol. In view of applying it to skin, retinol, retinol acetate, or retinol palmitate is preferable, and retinol acetate is most preferable.

The amount of vitamin A used in the present invention is not limited as long as a hyaluronidase inhibitory effect of rosemary extract can be enhanced. For example, vitamin A is usually included in a cosmetic or pharmaceutical product at 0.000001 - 0.01 w/w%, preferably 0.000005-0.001 w/w%, more preferably 0.00001 - 0.0001 w/w% in total.

Any mixing rate of rosemary extract and vitamin A can be used as long as a hyaluronidase inhibitory effect of rosemary extract can be enhanced. For example, 0.01 weight parts - 5 weight parts, preferably 0.05 weight parts - 1 weight parts, more preferably 0.1 weight parts - 0.5 weight parts of vitamin A can be used per 1 weight part of rosemary extract.

Since vitamin A does not usually have a hyaluronidase inhibitory activity, it can be said that a synergistic effect surpassing an additive effect is exerted by using vitamin A in combination with rosemary extract, if vitamin A could enhance the hyaluronidase inhibitory activity of rosemary extract. Even if vitamin A has a hyaluronidase inhibitory activity, the activity is weak, whereas vitamin A highly enhances the hyaluronidase inhibitory activity of rosemary extract when used in combination with rosemary extract. Therefore, even in such a case, a synergistic effect surpassing an additive effect is exerted by the combination of vitamin A and rosemary extract.

The hyaluronidase inhibitor of the present invention can be further contained in a cosmetic or pharmaceutical product. Therefore, in addition to rosemary extract and vitamin A, if necessary, an active ingredient and/or excipient, for example, a moisturizing agent, an antioxidant, an oily material, an UV protective agent, a surfactant, a thickening agent, a base ingredient, such as alcohols, glycerine, hyalronic acid, a pH modifier powder ingredient, an UV absorbing agent, a stabilizing agent, an antimicrobial agent, a flavoring agent, a colorant agent, water, several skin nutrition agents, etc., can be arbitrarily included in the cosmetic or pharmaceutical product, as long as the effect of the present invention is not impaired.

In addition, a sequestering agent, such as disodium edetate, trisodium edetate, sodium citrate, sodium polyphosphate, sodium metaphosphate, gluconic acid, etc., an antiseptic agent, such as methyl paraben, ethyl paraben, buthyl paraben, etc., a drug, such as caffeine, tannin, verapamil, tranexamic acid and a derivative thereof, licorice extract, glabridin, quince fruit hot water extract, various crude drugs, tocopherol acetate, glycyrrhizic acid and a derivative or salt thereof, a whitening agent, such as vitamin C, magnesium ascorbyl phosphate, ascorbic acid Glucoside, arbutin, Kojic acid, etc., saccharides, such as glucose, fructose, mannose, sucrose, treharose, etc., can also be arbitrarily included.

In addition, a cosmetic or pharmaceutical product comprising the hyaluronidase inhibitor of the present invention can be a skin external agent, such as a cosmetic product, a quasi-pharmaceutical product, etc., which is applied externally to the skin. Any formulations, such as solution system, a solubilized system, a emulsified system, a powder dispersal system, a water-oil bilayer system, a water-oil-powder trilayer system, an ointment, skin lotion, gel, aerozol, etc., can be applied.

A cosmetic product comprising the hyaluronidase inhibitor of the present invention includes, but is not limited to, a facial cosmetic product, such as skin lotion, milky lotion, cream, beauty serum, facial mask, a makeup cosmetic product, such as foundation, lip rouge, eye shadow, a sun protective agent, a perfumed cosmetic product, a hair cosmetic product, or a bath agent.

In addition to transdermal administration, the hyaluronidase inhibitor of the present invention can be administered orally, or parentally (for example, transmucossally, sublingually, intramuscularly, intra-articularly or subcutaneously). Therefore, in addition to a cosmetic or pharmaceutical product used for skin external use for preventing skin problems, such as wrinkled skin, rough skin, dry skin, dry-and-rough skin, acne or atopic dermatitis, the hyaluronidase inhibitor of the present invention can be included in food for preventing a reduction in hyaluronic acid in the body which is caused by aging, and a pharmaceutical composition, such as an agent for treating hyaluronic acid abnormal degradation disease, which is used for treating or preventing symptoms in which hyaluronic acid degradation is abnormally enhanced, in particular gingivitis, rheumatism, osteoarthritis, or arthritis, or is used for initial treatment of burn injury. When the hyaluronidase inhibitor of the present invention is used for a pharmaceutical composition, the formulation is not limited, but can be arbitrarily selected depending on an administration route. For example, a formulation suitable for oral administration includes a tablet, capsule, powder drug, fine grain drug, granule, liquid drug, syrup, etc., and a formulation suitable for parental administration includes an injectable drug, drops, suppository, inhalation, topical drug, transmucosal drug, adhesive patch, etc. The injectable drug can be used for any one of intraarticular injection, intravenous injection, intramuscular injection, subcutaneous injection, or drop infusion.

The present invention is directed to a non-therapeutic use of rosemary extract and retinyl acetate for reducing or preventing a decrease in hyaluronic acid, comprising applying rosemary extract and retinyl acetate to a subject suffering from a reduction in hyaluronic acid. Rosemary extract and retinyl acetate can be applied at the same time or separately at intervals. The non-therapeutic is directed to not only a method which is carried out individually, but also a method which is provided by a person other than a physician, such as a sales staff for cosmetic products or a beauty therapist, wherein the non-therapeutic use is provided as a prescription of a cosmetic product which is suitable for a customer at the time of providing a cosmetic product. The present invention is also directed to a method of non-therapeutic treatment for alleviating or preventing skin problems relating to a reduction of hyaluronic acid through inhibition of hyaluronidase activity, comprising applying rosemary extract and retinyl acetate to a subject suffering from a reduction in hyaluronic acid, where the skin problem can be selected from the group consisting of skin aging, wrinkled skin, rough skin and dry-and-rough skin.
The invention also relates to a composition comprising rosemary extract and vitamin A or a vitamin A derivative, which is selected from a group consisting of retinol, retinyl acetate and retinyl palmitate for use in alleviating or preventing skin problems selected from the group consisting of acne and atopic dermatitis, wherein hyaluronidase activity is inhibited.
In particular, said composition comprises rosemary extract and retinyl acetate.

In addition to a subject who is suffering from a a reduction in hyaluronic acid with aging, a subject suffering from a reduction in hyaluronic acid includes a subject who is suffering from a disease relating to a reduction in hyaluronic acid, such as atopic dermatitis, dry skin, arthritis, gingivitis, rheumatism, osteoarthritis, burn injury, etc.

### Examples

The present inventions are further explained in detail in the following Examples. However, the technical scope of the present invention should not be restricted by these examples. In addition, all percentages mean w/w%.

### Preparation Example: Preparation of Rosemary Extract

50 w/w% 1,3-butylene glycol aqueous solution was added to 10 kg of cut leaves of rosemary, and then the leaves were immersed for 7 to 10 days at room temperature. After the immersion, undesired substances were removed through filtration, and the filtrate was further incubated for 7 to 10 days in a cold location. After the incubation, undesired substances were removed through filtration so as to obtain rosemary extract BG.

### Example: An Examination for inhibiting hyaluronic acid degradation

50 µl hyaluronidase solution (1 mg/ml) (Sigma Aldrich Japan K.K.) was placed into a microtube, and 50 µ 1 of an evaluation drug was added, and then the mixture was incubated for 10 min at 37 °C. Rosemary extract BG (Maruzen Pharmaceuticals, Co., Ltd.), retinol acetate (BASF Japan Ltd.), and rosemary extract and retinol acetate were used as the evaluation drug, and water was used as a control instead of the evaluation agent. Rosemary extract BG was added so that the final concentration thereof after addition of hyaluronic acid sodium aqueous solution was 0.000165 w/w% at dry weight. Retinol acetate was added so that the final concentration thereof after addition of hyaluronic acid sodium aqueous solution was 0.0000516 w/w%. After incubation, 400 µl of 1 mg/ml sodium hyaluronate (Shiseido Co., Ltd., MW 1,100,000-1,600,000) was added to the solution, and then incubated at 37 °C, for 10 min, and then 50 µl of sodium hydroxide (0.2 M) was added to stop the reaction. This solution was boiled for 3 min, then cooled with flowing water. 300 µl of 10% p-DMAB (dimethylaminobenzaldehyde) solution (NACALAI TESQUE, Inc.) was added to the solution, and then incubated for 37 °C for 20 min. After bringing it back to room temperature, 100 µl of each solution was poured into each well of 96 well-plate, and OD 585 nm was determined by using POWERSCAN HT (DS Pharma Biomedical Co., Ltd.). The result wherein the absorbance in a control well was adjusted to be 100 was shown in Figure 1. Figure 1 showed that rosemary extract had a hyaluronidase inhibitory activity by itself, whereas retinol acetate did not have a hyaluronidase inhibitory activity, and that when rosemary extract was used in combination with retinol acetate, the hyaluronidase inhibitory activity of the rosemary extract was enhanced.

| Prescription Example (skin lotion) | (w/w %) |
|---|---|
| Ethanol | 5.0 |
| Glycerine | 0.5 |
| Dipropylene glycol | 2.0 |
| 1,3-butylene glycol | 6.0 |
| Sage oil | 0.01 |
| Citric acid | 0.02 |
| Sodium Citrate | 0.08 |
| Sodium hexametaphosphate | 0.03 |
| Hydroxy propyl-β-cyclodextrin | 0.1 |
| Retinol acetate | 0.00005 |
| Rosemary extract (dry weight) | 0.001 |
| Rosa Multiflora Fruit extract (dry weight) | 0.001 |
| Phellodendoron Amurense Bark extract (dry weight) | 0.001 |
| Rosa roxburghii fruit extract (dry weight) | 0.001 |
| Lavender oil | 0.1 |
| Prums Persica (Peach) Kernel Extract (dry weight) | 0.001 |
| Sodium arginate | 0.001 |
| Purified water | residue |

| Prescription Example (milky lotion) | (w/w %) |
|---|---|
| Dimethyl polysiloxane | 2.0 |
| Behenyl alcohol | 1.0 |
| Batyl Alcohol | 0.5 |
| Glycerine | 5.0 |
| 1,3-butylene glycol | 7.0 |
| Erythritol | 2.0 |
| Hardened oil | 3.0 |
| Scwaran | 6.0 |
| Tetra 2- ethylhexanoic acid pentaerythritol | 2.0 |
| Isostearic acid polyoxyethylene glycerol Mono Stearic acid polyoxyethylene glycerine | 1.0 |
| Retinol acetate | 0.0001 |
| Rosemary extract (dry weight) | 0.001 |
| Potassium hydroxide | ad lib. |
| hexametaphosphoric acid | 0.05 |
| Phenoxy ethanol | ad lib. |
| Carboxyvinyl polymer | 0.1 |
| Purified water | residue |

| Prescription Example (Cream) | (w/w %) |
|---|---|
| Liquid paraffin | 8.0 |
| Vaseline | 3.0 |
| Dimethyl polysiloxane | 2.0 |
| Stearyl alcohol | 3.0 |
| Behenyl alcoho | 2.0 |
| Glycerine | 5.0 |
| Dipropylene glycol | 4.0 |
| Trehalose | 1.0 |
| Tetra 2- ethylhexanoic acid pentaerythritol | 4.0 |
| Monoisostearic acid polyoxyethylene glycerol | 2.0 |
| Monostearic acid polyoxyethylene glycerine | 1.0 |
| Lipophilic monostearic acid glycerine | 2.0 |
| Citric acid | 0.05 |
| Sodium citrate | 0.05 |
| Potassium hydroxide | 0.015 |
| Oil soluble licorice extract (dry weight) | 8.0 |
| Retinol acetate | 0.0001 |
| Tocopherol acetate | 0.1 |
| Rosemary extract (dry weight) | 0.0002 |
| Paraoxybenzoic acid ester | ad lib. |
| Phenoxy ethanol | ad lib. |
| Dibutyl hydroxy toluene | ad lib. |
| Trisodium edetate | 0.05 |
| 4-t-butyl-4'-methoxydibenzoylmethan | 0.01 |
| Paramethoxy cinnamic acid 2-ethyl hexyl | 0.1 |
| β-carotene | 0.01 |
| Polyvinyl alcohol | 0.5 |
| Hydroxyethylcellulose | 0.5 |
| Carboxy vinyl polymer | 0.05 |
| Purified water | residue |
| Flavoring agent | ad lib. |

## Claims

1. The non-therapeutic use of rosemary extract and retinyl acetate, for alleviating or preventing skin problem relating to a reduction of hyaluronic acid through inhibition of hyaluronidase activity.

2. The non-therapeutic use according to claim 1, wherein the skin problem is selected from the group consisting of skin aging, wrinkled skin, rough skin, dry-and-rough skin.

3. A method of non-therapeutic treatment for alleviating or preventing skin problems relating to a reduction of hyaluronic acid through inhibition of hyaluronidase activity, comprising applying rosemary extract and retinyl acetate to a subject suffering from a reduction in hyaluronic acid.

4. The method according to claim 3, wherein the skin problem is selected from the group consisting of skin aging, wrinkled skin, rough skin, dry-and-rough skin.

5. A composition comprising rosemary extract and vitamin A or a vitamin A derivative, which is selected from a group consisting of retinol, retinyl acetate and retinyl palmitate for use in alleviating or preventing skin problems selected from the group consisting of acne and atopic dermatitis, wherein hyaluronidase activity is inhibited.

6. A composition for use according to claim 5 comprising rosemary extract and retinyl acetate.

## Patentansprüche

1. Nicht-therapeutische Verwendung von Rosmarinextrakt und Retinylacetat zur Linderung oder Vermeidung von Hautproblemen in Zusammenhang mit einer Reduktion von Hyaluronsäure durch Hemmung der Hyaluronidaseaktivität.

2. Nicht-therapeutische Verwendung nach Anspruch 1, wobei das Hautproblem ausgewählt ist aus der Gruppe bestehend aus Hautalterung, faltige Haut, raue Haut, trockene und raue Haut.

3. Verfahren der nicht-therapeutischen Behandlung zur Linderung oder Vermeidung von Hautproblemen in Zusammenhang mit einer Reduktion von Hyaluronsäure durch Hemmung der Hyaluronidaseaktivität, umfassend das Auftragen von Rosmarinextrakt und Retinylacetat bei einem Subjekt, das an einer Reduktion von Hyaluronsäure leidet.

4. Verfahren nach Anspruch 3, wobei das Hautproblem ausgewählt ist aus der Gruppe bestehend aus Hautalterung, faltige Haut, raue Haut, trockene und raue Haut.

5. Zusammensetzung umfassend Rosmarinextrakt und Vitamin A oder ein Vitamin-A-Derivat, welches ausgewählt ist aus der Gruppe bestehend aus Retinol, Retinylacetat und Retinylpalmitat für die Verwendung zur Linderung oder Vermeidung von Hautproblemen, ausgewählt aus der Gruppe bestehend aus Akne und atopischer Dermatitis, wobei die Hyaluronidaseaktivität gehemmt ist.

6. Zusammensetzung zur Verwendung nach Anspruch 5 umfassend Rosmarinextrakt und Retinylacetat.

## Revendications

1. Utilisation non thérapeutique d'extrait de romarin et d'acétate de rétinyle, pour atténuer ou prévenir un problème de peau lié à une réduction de l'acide hyaluronique par inhibition de l'activité hyaluronidase.

2. Utilisation non thérapeutique selon la revendication 1, dans laquelle le problème de peau est choisi dans le groupe constitué par un vieillissement de la peau, une peau ridée, une peau rêche, une peau sèche et rêche.

3. Méthode de traitement non thérapeutique pour atténuer ou prévenir des problèmes de peau liés à une réduction de l'acide hyaluronique par inhibition de l'activité de hyaluronidase, comprenant l'application d'extrait de romarin et d'acétate de rétinyle à un sujet souffrant d'une réduction de l'acide hyaluronique.

4. Méthode selon la revendication 3, dans laquelle le problème de peau est choisi dans le groupe constitué par un vieillissement de la peau, une peau ridée, une peau rêche, une peau sèche et rêche.

5. Composition comprenant de l'extrait de romarin et de la vitamine A ou un dérivé de vitamine A, qui est choisi dans le groupe constitué par le rétinol, l'acétate de rétinyle et le palmitate de rétinyle, pour une utilisation dans l'atténuation ou la prévention de problèmes de peau choisis dans le groupe constitué par l'acné et la dermite atopique, dans laquelle l'activité hyaluronidase est inhibée.

6. Composition pour une utilisation selon la revendication 5, comprenant de l'extrait de romarin et de l'acétate de rétinyle.
